# EUROPEAN PATENT SPECIFICATION

(11) **EP 2 854 924 B1**
(45) Date of publication and mention of the grant of the patent: **03.10.2018**
(21) Application number: 13731534.7
(22) Date of filing: 23.05.2013
(51) Int. Cl.: A61B 17/34, A61B 17/22, A61M 25/01, A61B 17/3207

(54) **SUBINTIMAL RE-ENTRY CATHETER WITH AN EXPANDABLE STRUCTURE**
SUBINTIMALER RE-ENTRY-KATHETER MIT ERWEITERBARER STRUKTUR
CATHÉTER DE RÉINTRODUCTION SOUS-INTIMALE AVEC UNE STRUCTURE DILATABLE

(30) Priority: 24.05.2012 US 201261651262 P
(43) Date of publication of application: 08.04.2015
(73) Proprietor: Boston Scientific Scimed, Inc., Maple Grove, MN 55311-1566 (US)
(72) Inventor: ZHOU, Pu, Maple Grove, Minnesota 55311 (US); WANG, Huisun, Maple Grove, Minnesota 55311 (US)
(74) Representative: Vossius & Partner Patentanwälte Rechtsanwälte mbB
(86) International application number: PCT/US2013/042398
(87) International publication number: WO 2013/177383

(56) References cited:
- US-A1- 2005 149 062
- US-A1- 2008 243 065
- US-A1- 2009 209 910
- US-A1- 2010 063 534

## Description

### TECHNICAL FIELD

The disclosure is directed to devices for recanalization of an occluded blood vessel. More particularly, the disclosure is directed to devices
for re-entry into the true lumen from the extraluminal or subintimal space of a blood vessel.

### BACKGROUND

Chronic total occlusion (CTO) is an arterial vessel blockage that obstructs blood flow through the vessel, and can occur in both coronary and peripheral arteries. In some instances, it may be difficult or impossible to pass through the CTO with a medical device in an antegrade direction to recanalize the vessel. Accordingly, techniques have been developed for creating a subintimal pathway (i.e., a pathway between the intimal and adventitial tissue layers of the vessel) around the occlusion and then re-entering the true lumen of the vessel distal of the occlusion in an attempt to recanalize the vessel. In some instances re-entering the true lumen from the subintimal space and/or recanalization can be difficult. Accordingly, it is desirable to provide alternative recanalization devices and/or methods of recanalizing a blood vessel in which a CTO is present.

US 2008/243065 discloses a method of introducing a catheter between two layers of a wall of a body lumen. The catheter includes a balloon disposed at a distal end thereof. The catheter is formed with a first lumen through the balloon for passing therethrough a guidewire and a second lumen for inflation and deflation of the balloon. The method includes expanding the balloon so that a distal portion of the first lumen that passes through the balloon is bent with respect to a portion of the first lumen proximal to the balloon and is directed towards the body lumen.

US 2005/149062 describes a method for puncturing a proximal membrane without puncturing a second, distal membrane. The method comprises distally advancing an elongated body through an elongated tubular member to a first position where a distal end of the elongated body is in a first configuration. In the first configuration, the distal end of the elongated body is positioned outside the distal end of the elongated tubular member. The distal end of the elongated body has a distal tip, in this first configuration, that is sufficiently rigid and sharp to puncture the first, proximal membrane. From this first configuration, the distal tip of the elongated body is slightly advanced to puncture the first, proximal membrane. After puncturing the first, proximal membrane, the elongated body is advanced through the punctured proximal membrane whereby the distal tip of the elongated body moves into a second configuration. In the second configuration, the distal tip of the elongated body curves into a j-shape, forming a blunt distal surface facing the second, distal membrane.

US 2009/209910 relates to a method of facilitating treatment via a vascular wall defining a vascular lumen containing an occlusion therein. The method may include providing a first intravascular device having a distal portion and at least one aperture and positioning the distal portion of the first intravascular device in the vascular wall. The method may further include providing a reentry device having a body and a distal tip, the distal tip having a natural state and a compressed state and inserting the distal tip, in the compressed state, in the distal portion of the first intravascular device. The method may further include advancing the distal tip, in the natural state, through the at least one aperture of the first intravascular device.

US 2010/0063534 A1 discloses a subintimal crossing device comprising a sheath, a shaft, an expandable element and a pull wire. The distal end of the expandable element is closed and comprises an atraumatic tip in form of a weld ball. The pull wire is attached to the distal end of the expandable element and extends proximally through the lumen of the shaft. The expandable element may be expanded by pushing on the shaft and pulling on a pull wire.

### SUMMARY

The disclosure is directed to several alternative designs, materials arid methods of manufacturing medical device structures and assemblies, and uses thereof.

Accordingly, one illustrative embodiment is a subintimal recanalization catheter assembly for recanalizing a blood vessel having an occlusion in a lumen thereof. The catheter assembly includes an outer tubular member and an inner tubular member. The outer tubular member has a lumen extending therethrough and the inner tubular member has a lumen extending therethrough. The inner tubular member is disposed in the lumen of the outer tubular member and movable relative to the outer tubular member. An expandable structure is secured to a distal portion of the inner tubular member. The expandable structure is configured to be actuated between a collapsed configuration and an expanded configuration by longitudinal movement of the inner tubular member relative to the outer tubular member. The assembly also includes a penetration member sized to be advanced through the lumen of the inner tubular member such that a distal portion of the penetration member is extendable distally of a distal end of the inner tubular member. The catheter assembly is configured to be advanced into a subintimal space between a first tissue layer and a second tissue layer of a wall of the blood vessel to facilitate reentry into the lumen of the blood vessel by advancing the penetration member distally out of the lumen of the inner tubular member and through the first tissue layer into the lumen of the blood vessel distal of the occlusion.

Another illustrative embodiment is a subintimal recanalization catheter assembly for recanalizing a blood vessel having an occlusion in a lumen thereof. The catheter assembly includes a hub assembly, an outer tubular member extending distally from the hub assembly, and an inner tubular member extending distally from the hub assembly through a lumen of the outer tubular member. The catheter assembly also includes an expandable structure having a distal end secured to a distal portion of the inner tubular member and a proximal end secured to a distal portion of the outer tubular member. The expandable structure is configured to be actuated between a collapsed configuration and an expanded configuration by longitudinal movement of the inner tubular member relative to the outer tubular member. Additionally, the assembly includes a penetration member sized to be advanced through the lumen of the inner tubular member such that a distal portion of the penetration member is extendable distally of a distal end of the inner tubular member. The catheter assembly is configured to be advanced into a subintimal space between a first tissue layer and a second tissue layer of a wall of the blood vessel to facilitate reentry into the lumen of the blood vessel by advancing the penetration member distally out of the lumen of the inner tubular member and through the first tissue layer into the lumen of the blood vessel distal of the occlusion.

Yet another illustrative example not being part of the invention is a method of recanalizing a blood vessel having an occlusion in a lumen thereof. The method includes advancing a catheter including an expandable structure positioned in a collapsed configuration on a distal end thereof into a subintimal space between a first tissue layer and a second tissue layer of a wall of a vessel such that the expandable structure is positioned in the subintimal space distal to the occlusion. An inner tubular member of the catheter is then moved longitudinally relative to an outer tubular member of the catheter to expand the expandable structure to an expanded configuration within the subintimal space. Expanding the expandable structure to the expanded configuration causes a distal portion of the inner tubular member extending through the expandable structure to deflect toward the lumen of the blood vessel. Thereafter, a penetration member is advanced through the inner tubular member such that the penetration member extends distally from the inner tubular member through the first tissue layer into the lumen of the blood vessel.

The above summary of some example embodiments is not intended to describe each disclosed embodiment or every implementation of the aspects of the disclosure.

### BRIEF DESCRIPTION OF THE DRAWINGS

The aspects of the disclosure may be more completely understood in consideration of the following detailed description of various embodiments in connection with the accompanying drawings, in which:
FIG. 1 is a side plan view of an exemplary catheter apparatus for recanalization of a blood vessel in a collapsed configuration;
FIG. 2 is side plan view of the exemplary catheter apparatus of FIG. 1 in an expanded configuration;
FIG. 3 is a cross-sectional view of the catheter apparatus of FIG. 2 taken along line 3-3;
FIG. 4 is a cross-sectional view of the catheter apparatus of FIG. 2 taken along line 4-4;
FIG. 5 is a side plan view of an another exemplary catheter apparatus for recanalization of a blood vessel in a collapsed configuration;
FIG. 6 is side plan view of the exemplary catheter apparatus of FIG. 5 in an expanded configuration;
FIG. 7 is a cross-sectional view of the catheter apparatus of FIG. 6 taken along line 7-7;
FIG. 8 is a cross-sectional view of the catheter apparatus of FIG. 6 taken along line 8-8;
FIG. 9 is a side view of an exemplary expandable structure in an expanded configuration;
FIG. 10 is a side view of another exemplary expandable structure in an expanded configuration;
FIGS. 11A, 11B and 11C illustrate exemplary configurations of a predefined bending location along a strut of an expandable structure;
FIG. 12 illustrates an exemplary expandable structure in an expanded configuration in a subintimal space of a blood vessel; and
FIGS. 13-17 illustrate aspects of an exemplary method for recanalizing an occluded blood vessel using the catheter apparatus of FIG. 1.

While the aspects of the disclosure are amenable to various modifications and alternative forms, specifics thereof have been shown by way of example in the drawings and will be described in detail. It should be understood, however, that the intention is not to limit aspects of the disclosure to the particular embodiments described. The subject-matter of the invention is defined by the appended claims.

### DETAILED DESCRIPTION

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (i.e., having the same function or result). In many instances, the term "about" may be indicative as including numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

Although some suitable dimensions, ranges and/or values pertaining to various components, features and/or specifications are disclosed, one of skill in the art, incited by the present disclosure, would understand desired dimensions, ranges and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The detailed description and the drawings, which are not necessarily to scale, depict illustrative embodiments and are not intended to limit the scope of the disclosure. The illustrative embodiments depicted are intended only as exemplary. Selected features of any illustrative embodiment may be incorporated into an additional embodiment unless clearly stated to the contrary.

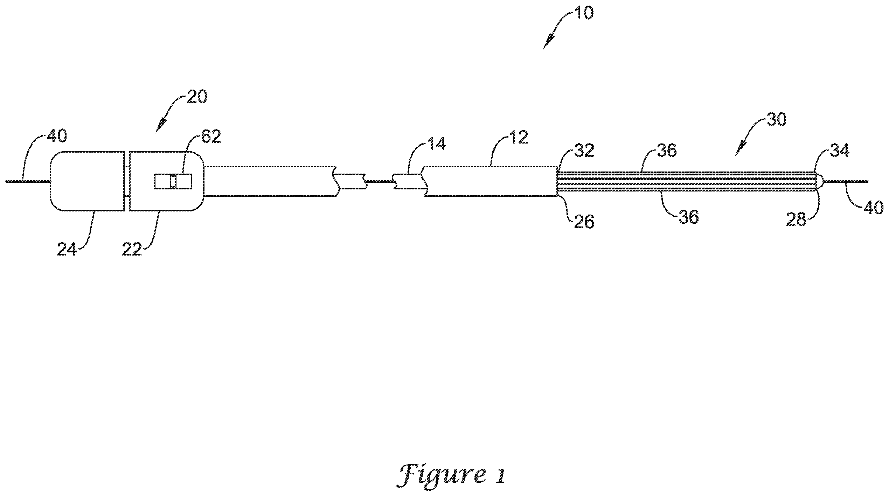

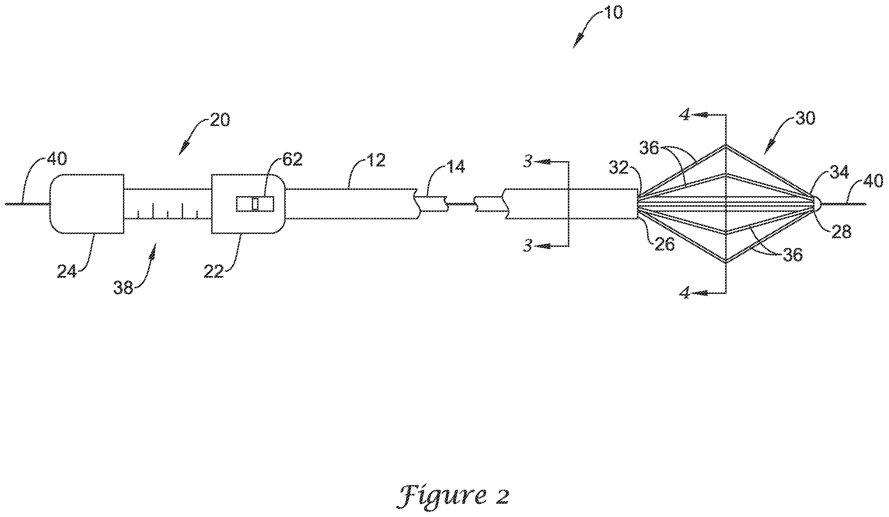

An exemplary recanalization catheter 10 is illustrated at FIGS. 1 and 2 in a collapsed configuration and an expanded configuration, respectively. The recanalization catheter 10 may include an outer tubular member 12 having a lumen 16 extending therethrough and an inner tubular member 14 having a lumen 18 extending therethrough. The inner tubular member 14 may be disposed in the lumen 16 of the outer tubular member 12 and movable relative to the outer tubular member 12. For example, the inner tubular member 14 may be rotatable and/or movable longitudinally relative to the outer tubular member 12.

The recanalization catheter 10 may include an actuation mechanism configured to actuate the inner tubular member 14 longitudinally and/or rotationally relative to the outer tubular member 12. For example, in some embodiments the recanalization catheter 10 may include a handle or hub assembly 20 configured to be manipulated by a user to effect longitudinal and/or rotational movement between the inner tubular member 14 and the outer tubular member 12. In some instances, the hub assembly 20 may include a first hub 22 secured to the proximal end of the outer tubular member 12 and a second hub 24 secured to the proximal end of the inner tubular member 14. The first hub 22 may be rotatable and/or longitudinally translatable relative to the second hub 24 to effect movement of the inner tubular member 14 relative to the outer tubular member 12. The inner tubular member 14 may extend from the hub assembly 20 through the lumen 16 of the outer tubular member 12 such that the distal end 28 of the inner tubular member 14 is positioned distal of the distal end 26 of the outer tubular member 12. Thus, actuation of the inner tubular member 14 relative to the outer tubular member 12 may alter the distance between the distal end 26 of the outer tubular member 12 and the distal end 28 of the inner tubular member 14. In some instances, the actuation mechanism may be configured to provide pure longitudinal movement of the inner tubular member 14 relative to the outer tubular member 12, while in other instances, the actuation mechanism may be configured to provide longitudinal movement of the inner tubular member 14 relative to the outer tubular member 12 via rotational movement of the inner tubular member 14 relative to the outer tubular member 12. In other words, in some embodiments, the inner tubular member 14 may be rotatable relative to the outer tubular member 12 to produce longitudinal movement of the inner tubular member 14 relative to the outer tubular member 12. For example, a threaded region between the inner tubular member 14 and the outer tubular member 12 may produce longitudinal movement via rotational movement. Other actuation mechanisms are contemplated. For example, in other instances, the hub assembly 20 may include a lever, button, knob, or other actuatable member to actuate the inner tubular member 14 relative to the outer tubular member 12.

The recanalization catheter 10 may include an expandable structure 30, such as an expandable cage, scaffold, or framework, secured at a distal portion, thereof. In some instances, the expandable structure 30 may be an expandable cage or framework formed of one or more, or a plurality of struts 36 which may be automatically or manually expanded, or other manually expandable or automatically expandable structure. The expandable structure 30 may have advantages over prior art devices utilizing an inflatable balloon mounted to a catheter shaft. For example, the expandable structure 30 may be constructed to have a smaller collapsed or delivery configuration than a folded balloon on a catheter shaft and/or expansion of the expandable structure 30 may be incrementally controlled by a user.

The expandable structure 30 may be formed from any number of biocompatible materials, including polymeric materials, metals, and metal alloys, such as stainless steel, tantalum, or a nickel titanium alloy such as a superelastic nickel titanium alloy known as Nitinol, which may have shape memory properties in some instances. In some embodiments, the expandable structure 30 may be formed of a radiopaque material, or have radiopaque properties to make the expandable structure 30 visible under fluoroscopy during a medical procedure. For example, in some instances the expandable structure 30 may be formed of a radiopaque metal or alloy such as tungsten, platinum or a platinum-chromium alloy, molybdenum or a molybdenum alloy, or a polymer having a radiopaque filler dispersed therein.

In some instances, the expandable structure 30 may be secured to a distal portion of the inner tubular member 14 and/or the outer tubular member 12. The expandable structure 30 may be expandable from a collapsed, delivery configuration shown in FIG. 1 to an expanded configuration shown in FIG. 2. For example, the expandable structure 30 may be manually expandable from the collapsed configuration to the expanded configuration by rotational and/or longitudinal movement of the inner tubular member 14 relative to the outer tubular member 12. In the embodiment of FIGS. 1 and 2, a proximal end 32 of the expandable structure 30 may be fixedly secured to a distal portion of the outer tubular member 12 proximate the distal end 26 of the outer tubular member 12, and a distal end 34 of the expandable structure 30 may be fixedly secured to a distal portion of the inner tubular member 14 proximate the distal end 28 of the inner tubular member 14 with a distal portion of the inner tubular member 14 extending through the expandable structure 30. Accordingly, longitudinal movement of the inner tubular member 14 proximally relative to the outer tubular member 12 may cause the distal end 34 of the expandable structure 30 to move toward the proximal end 32 of the expandable structure 30 to expand the expandable structure 30. At the conclusion of a medical procedure, longitudinal movement of the inner tubular member 14 distally relative to the outer tubular member 12 may cause the distal end 34 of the expandable structure 30 to move away from the proximal end 32 of the expandable structure 30 to collapse the expandable structure 30 back to the collapsed configuration for withdrawal from the blood vessel.

The handle assembly 20 may include a locking mechanism 62 to selectively lock the inner tubular member 14 from longitudinal movement relative to the outer tubular member 12 in the collapsed configuration, in the expanded configuration, and/or at one or more or at one of a plurality of positions between the collapsed configuration and the expanded configuration. For example, the locking mechanism 62 may be a lever, button, cam, threading or other mechanism configured to selectively prevent relative movement between the inner tubular member 14 and the outer tubular member 12, as desired. For instance, the locking mechanism 62 may be configured to be selectively brought into contact with and bear against the inner tubular member 14 to provide sufficient frictional engagement to prevent movement of the inner tubular member 14.

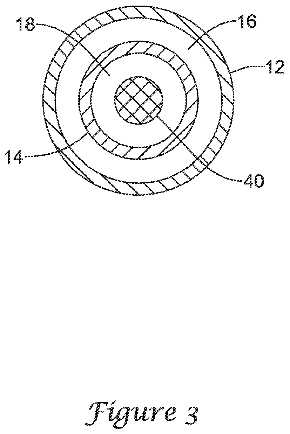

The recanalization catheter 10 may be configured to be advanced over a guidewire 40 for delivery to a remote location in the vasculature of a patient. For example, in some instances the lumen 18 of the inner tubular member 14 of the catheter 10 may be a guidewire lumen configured to receive the guidewire 40 therethrough. FIG. 3 is a cross-sectional view of the catheter 10 illustrating an exemplary arrangement of the guidewire 40, the inner tubular member 14, and the outer tubular member 12. As shown, the inner tubular member 14 may extend through the lumen 16 of the outer tubular member 12, while the guidewire 40 may extend through the lumen 18 of the inner tubular member 14. In instances in which the catheter 10 may be configured as an over-the-wire (OTW) catheter, the guidewire lumen may extend through the entire length of the catheter 10 from a distal port at a distal end of the inner tubular member 14 to a proximal guidewire port in the hub assembly 20. In other instances in which the catheter 10 may be configured as a single-operator-exchange (SOE), the guidewire lumen may extend through a distal portion of the catheter 10 from a distal port at a distal end of the inner tubular member 14 to a proximal guidewire port located distal of the hub assembly 20.

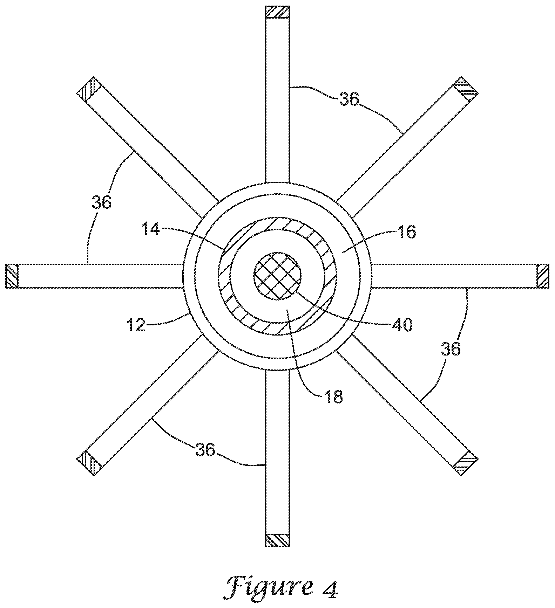

The expandable structure 30, such as an expandable cage, scaffold, or framework, may have any desired configuration configured to be automatically or manually expanded from a collapsed configuration. The cross-sectional view of FIG. 4 illustrates one possible configuration in which the expandable structure 30 may be an open structure having a plurality of circumferentially arranged longitudinal struts 36 extending radially outward from the longitudinal axis of the catheter 10 and circumferentially arranged around the longitudinal axis of the catheter 10. In other instances, the struts 36 may extend helically around the longitudinal axis or in another manner, as desired.

The recanalization catheter 10 may also include an indicator configured to indicate to what extent the expandable structure 30 is expanded to. For instance, the hub assembly 20 may include an indicator 38 including one or more, or a plurality of markings. As shown in FIG. 2, in some instances the indicator 38 may be a series of graduated lines, extending along a portion of the inner tubular member 14 or a portion of the hub assembly 20, Accordingly, the indicator 38 may provide a visual indication of the extent that the inner tubular member 14 is moved longitudinally relative to the outer tubular member 12, which may be directly related to the extent to which the expandable structure has expanded from the collapsed configuration to the expanded configuration. For example, as the second hub 24 attached to the inner tubular member 14 is moved proximally, away from the first hub 22 attached to the outer tubular member 12, the graduated lines of the indicator 38 may be exposed, and thus visible to a user to provide visual feedback to the user at the proximal end of the catheter 10 regarding the amount of relative longitudinal movement between the inner tubular member 14 and the outer tubular member 12, and thus the extent or state of expansion of the expandable structure 30. In some instances, the indicator 38 may be utilized by the medical personnel to incrementally expand the expandable structure 30 to a desired expanded state.

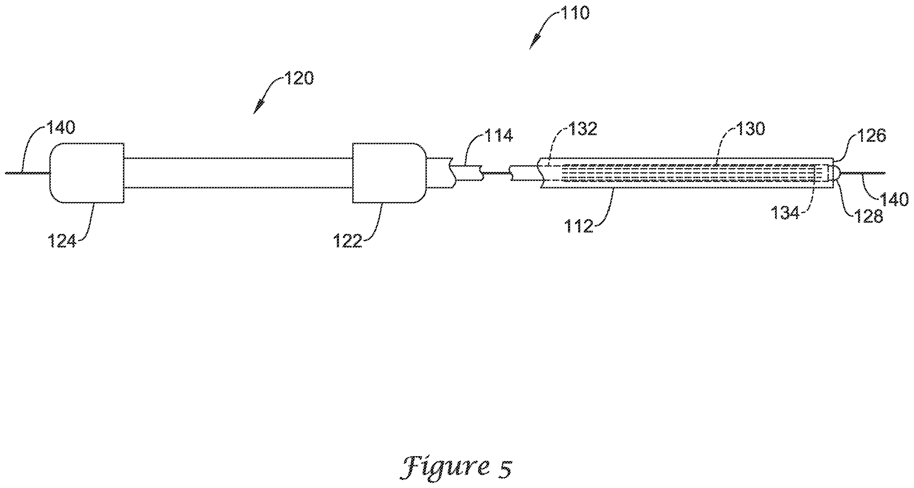

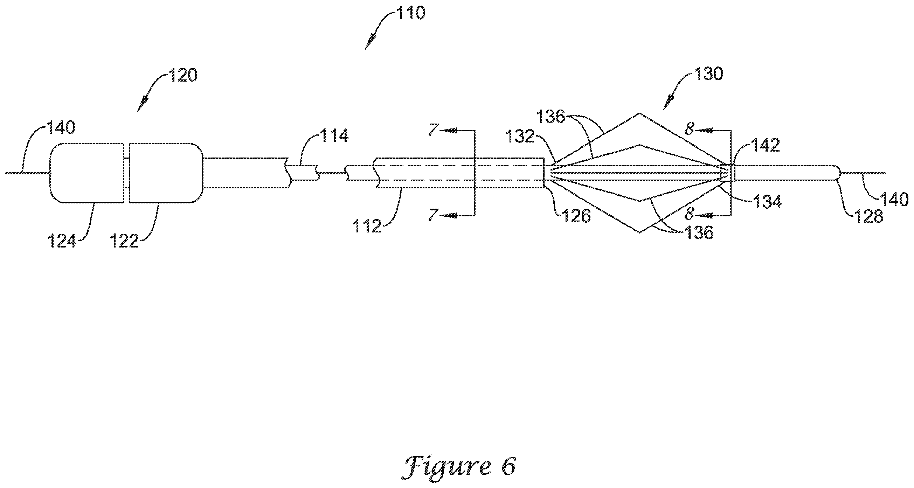

Another exemplary recanalization catheter 110 is illustrated at FIGS. 5 and 6 in a collapsed configuration and an expanded configuration, respectively. The recanalization catheter 110 may be similar to the recanalization catheter 10 in many respects. For example, the recanalization catheter 110 may include an outer tubular member 112 having a lumen 116 extending therethrough and an inner tubular member 114 having a lumen 118 extending therethrough. The inner tubular member 114 may be disposed in the lumen 116 of the outer tubular member 112 and movable relative to the outer tubular member 112. For example, the inner tubular member 114 may be rotatable and/or movable longitudinally relative to the outer tubular member 112.

The recanalization catheter 110 may include an actuation mechanism configured to actuate the inner tubular member 114 longitudinally and/or rotationally relative to the outer tubular member 112. For example, in some embodiments the recanalization catheter 110 may include a handle or hub assembly 120 configured to be manipulated by a user to effect longitudinal and/or rotational movement between the inner tubular member 114 and the outer tubular member 112. In some instances, the hub assembly 120 may include a first hub 122 secured to the proximal end of the outer tubular member 112 and a second hub 124 secured to the proximal end of the inner tubular member 114. The first hub 122 may be rotatable and/or longitudinally translatable relative to the second hub 124 to effect movement of the inner tubular member 14 relative to the outer tubular member 112. The inner tubular member 114 may extend from the hub assembly 120 through the lumen 116 of the outer tubular member 112 such that the distal end 128 of the inner tubular member 114 is positionable distal of the distal end 126 of the outer tubular member 112. Thus, actuation of the inner tubular member 114 relative to the outer tubular member 112 may alter the distance between the distal end 126 of the outer tubular member 112 and the distal end 128 of the inner tubular member 114. In some instances, the actuation mechanism may be configured to provide pure longitudinal movement of the inner tubular member 114 relative to the outer tubular member 112, while in other instances, the actuation mechanism may be configured to provide longitudinal movement of the inner tubular member 114 relative to the outer tubular member 112 via rotational movement of the inner tubular member 114 relative to the outer tubular member 112. In other words, in some embodiments, the inner tubular member 114 may be rotatable relative to the outer tubular member 112 to produce longitudinal movement of the inner tubular member 114 relative to the outer tubular member 112. For example, a threaded region between the inner tubular member 114 and the outer tubular member 112 may produce longitudinal movement via rotational movement. Other actuation mechanisms are contemplated. For example, in other instances, the hub assembly 120 may include a lever, button, knob, or other actuatable member to actuate the inner tubular member 114 relative to the outer tubular member 112.

The recanalization catheter 110 may include an expandable structure 130, such as an expandable cage, scaffold, or framework, secured at a distal portion, thereof. Similarly to the expandable structure 30, in some instances, the expandable structure 130 may be an expandable cage or framework formed of one or more, or a plurality of struts 136 which may be automatically or manually expanded, or other manually expandable or automatically expandable structure. The expandable structure 130 may have advantages over prior art devices utilizing an inflatable balloon mounted to a catheter shaft. For example, the expandable structure 130 may be constructed to have a smaller collapsed or delivery configuration than a folded balloon on a catheter shaft and/ the expandable structure 130 may be automatically expanded without the need for inflation fluid.

The expandable structure 130 may be formed from any number of biocompatible materials, including polymeric materials, metals, and metal alloys. The expandable structure 130 may be formed of a shape memory material such that the expandable structure 130 automatically expands to an expanded configuration from a collapsed configuration when unconstrained. For example, the expandable structure 130 may be formed of a shape member alloy, such as a superelastic nickel titanium alloy known as Nitinol, or a shape memory polymer, or other shape memory material which may have shape memory properties in some instances. In some embodiments, the expandable structure 130 may be formed of a radiopaque material, or have radiopaque properties to make the expandable structure 130 visible under fluoroscopy during a medical procedure. For example, in some instances the expandable structure 130 may include portions, such as radiopaque markers formed of a radiopaque metal or alloy such as tungsten, platinum or a platinum-chromium alloy, molybdenum or a molybdenum alloy, or a polymer having a radiopaque filler dispersed therein.

In some instances, the expandable structure 130 may be secured to a distal portion of the inner tubular member 114 to be deployed from the lumen 116 of the outer tubular member 112 upon actuation of the inner tubular member 114 relative to the outer tubular member 112. For example, the expandable structure 130 may be expandable from a collapsed, delivery configuration shown in FIG. 5, in which the expandable structure 130 is disposed and constrained in the lumen 116 of the outer tubular member 112 in a collapsed state, to an expanded configuration shown in FIG. 6, in which the expandable structure 130 is deployed from the lumen 116 of the outer tubular member 112 to freely expand to an expanded state. For example, the expandable structure 130 may be automatically expandable from the collapsed configuration to the expanded configuration upon expelling the expandable structure 130 from within the outer tubular member 112. In some instances the expandable structure 130 may be formed of a resilient material or a shape memory material biased to return to its expanded configuration when unconstrained.

In the embodiment of FIGS. 5 and 6, a proximal end 132 of the expandable structure 130 may be fixedly secured to a distal portion of the inner tubular member 114 and a distal end 134 of the expandable structure 130 may be slidably coupled to the distal portion of the inner tubular member 114 proximate the distal end 128 of the inner tubular member 114. For example, the distal end 134 of the expandable structure 130 may include a slidable ring 142 slidably disposed around the inner tubular member 114. In some embodiments, the distal ends of the struts 136 of the expandable structure 130 may be secured to the slidable ring 142. Accordingly, when the expandable structure 130 is not constrained by the outer tubular member 112, the slidable ring 142, and thus the distal end 134 of the expandable structure 130 may move towards the proximal end 132 of the expandable structure 130 to expand the expandable structure 130.

To deploy the expandable structure 130 from the outer tubular member 112, the second hub 124 may be actuated distally relative to the first hub 122, thus moving the inner tubular member 114 distally relative to the outer tubular member 112 until the expandable structure 130 is expelled from the lumen 116 of the outer tubular member 112. Once unconstrained by the outer tubular member 112, the expandable structure 130 may automatically radially expand to the expanded configuration.

At the conclusion of a medical procedure, longitudinal movement of the inner tubular member 114 proximally relative to the outer tubular member 112 may press the expandable structure 130 against the distal end 126 of the outer tubular member 112 to urge the expandable structure 130 back to the collapsed configuration, and thus move the distal end 134 away from the proximal end 132 to collapse the expandable structure 130 for removal from the blood vessel. It is noted that in some embodiments, the distal end 134 of the expandable structure 130 may be fixedly secured to the inner tubular member 114, while the proximal end 132 of the expandable structure 130 may be slidably disposed on the inner tubular member 114, in a similar fashion.

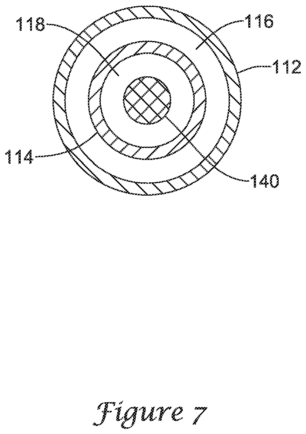

The recanalization catheter 110 may be configured to be advanced over a guidewire 140 for delivery to a remote location in the vasculature of a patient. For example, in some instances the lumen 118 of the inner tubular member 114 of the catheter 110 may be a guidewire lumen configured to receive the guidewire 140 therethrough. FIG. 7 is a cross-sectional view of the catheter 110 illustrating an exemplary arrangement of the guidewire 140, the inner tubular member 114 and the outer tubular member 112. As shown, the inner tubular member 114 may extend through the lumen 116 of the outer tubular member 112, while the guidewire 140 may extend through the lumen 118 of the inner tubular member 114. In instances in which the catheter 110 may be configured as an over-the-wire (OTW) catheter, the guidewire lumen may extend through the entire length of the catheter 110 from a distal port at a distal end of the inner tubular member 114 to a proximal guidewire port in the hub assembly 120. In other instances in which the catheter 110 may be configured as a single-operator-exchange (SOE), the guidewire lumen may extend through a distal portion of the catheter 110 from a distal port at a distal end of the inner tubular member 114 to a proximal guidewire port located distal of the hub assembly 120.

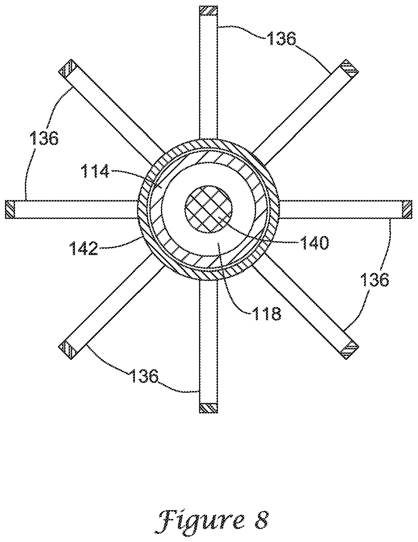

The expandable structure 130, such as an expandable cage, scaffold, or framework, may have any desired configuration configured to be automatically or manually expanded from a collapsed configuration. The cross-sectional view of FIG. 8 illustrates one possible configuration in which the expandable structure 130 may be an open structure having a plurality of circumferentially arranged longitudinal struts 136 extending radially outward from the longitudinal axis of the catheter 110 and circumferentially arranged around the longitudinal axis of the catheter 110. In other instances, the struts 136 may extend helically around the longitudinal axis or in another manner, as desired. The struts 136 may be secured to the slidable ring 142 which is slidably disposed around the inner tubular member 114.

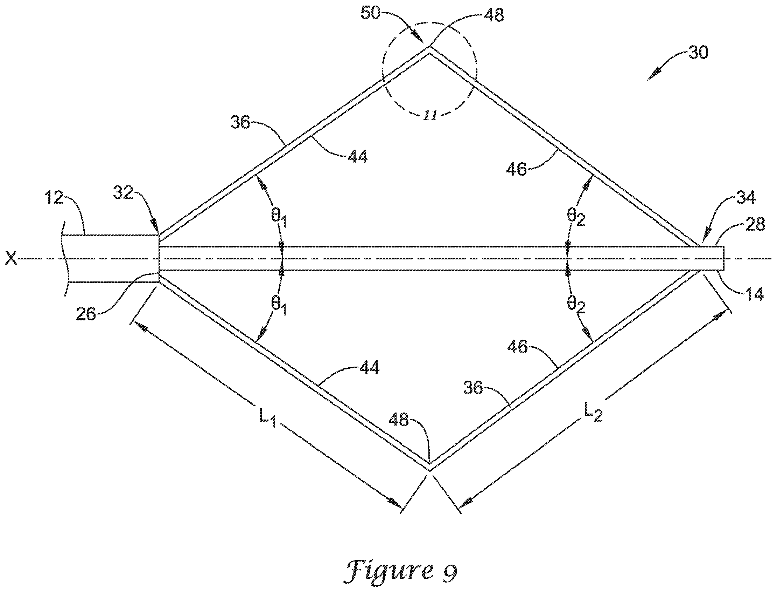

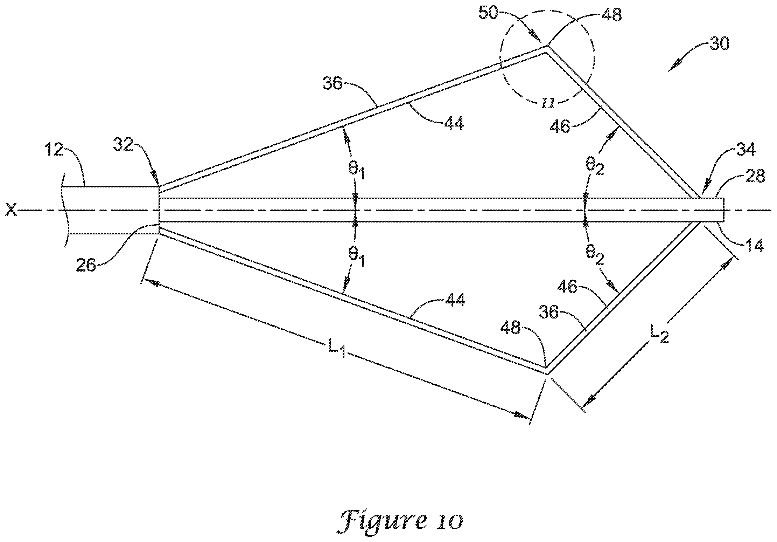

FIGS. 9 and 10 illustrate exemplary embodiments of struts 36 of an expandable structure 30. Although discussion is directed to the expandable structure 30, it is noted that aspects of the exemplary configurations may also be manifested in the expandable structure 130, as desired.

Referring to FIG. 9, one exemplary embodiment of the expandable structure 30 may include a plurality of struts 36 extending from the proximal end 32 of the expandable structure 30 to the distal end 34 of the expandable structure 30. Each strut 36 may include a proximal portion 44 and a distal portion 46. The proximal portion 44 of each strut 36 may extend radially outward and distally from the proximal end 32 and thus the distal end 26 of the outer tubular member 12 to a radially outermost extent 48 of the expandable structure 30, while the distal portion 46 of each strut may extend radially inward and distally from the outermost extent 48 of the expandable structure 30 to the distal end 34 and thus the distal end 28 of the inner tubular member 14. In the embodiment of FIG. 9, the proximal portion 44 may have a length L₁ and the distal portion 46 may have a length L₂ substantially equal to the length L₁ of the proximal portion 44. Accordingly, in the expanded configuration, the proximal portion 44 may extend at an angle θ₁ from the central longitudinal axis X of the catheter 10 (e.g., the central longitudinal axis of the outer tubular member 12) and the distal portion 46 may extend at an angle θ₂ from the central longitudinal axis X of the catheter 10 (e.g., the central longitudinal axis of the outer tubular member 12) substantially equal to the angle θ₁.

In other embodiments, such as illustrated in FIG. 10, the proximal portion 44 may have a length L₁ and the distal portion 46 may have a length L₂ substantially less than the length L₁ of the proximal portion 44. In other words, the length L₁ of the proximal portion 44 may be greater than the length L₂ of the distal portion 46. Accordingly, in the expanded configuration, the proximal portion 44 may extend at an angle θ₁ from the central longitudinal axis X of the catheter 10 (e.g., the central longitudinal axis of the outer tubular member 12) and the distal portion 46 may extend at an angle θ₂ from the central longitudinal axis X of the catheter 10 (e.g., the central longitudinal axis of the outer tubular member 12) greater than the angle θ₁.

The angle θ₁ of the proximal portions 44 of the struts 36 of the expandable structure 30 in the expanded configuration may dictate the extent that the distal portion of the inner tubular member 14 bends or deflects from the central longitudinal axis X of the outer tubular member 12 when expanded within a subintimal space between adjacent tissue layers during a recanalization procedure, as discussed further herein. In some embodiments, the angle θ₁ may be in the range of about 25 degrees to about 75 degrees, about 30 degrees to about 60 degrees, about 40 degrees to about 50 degrees, or about 45 degrees.

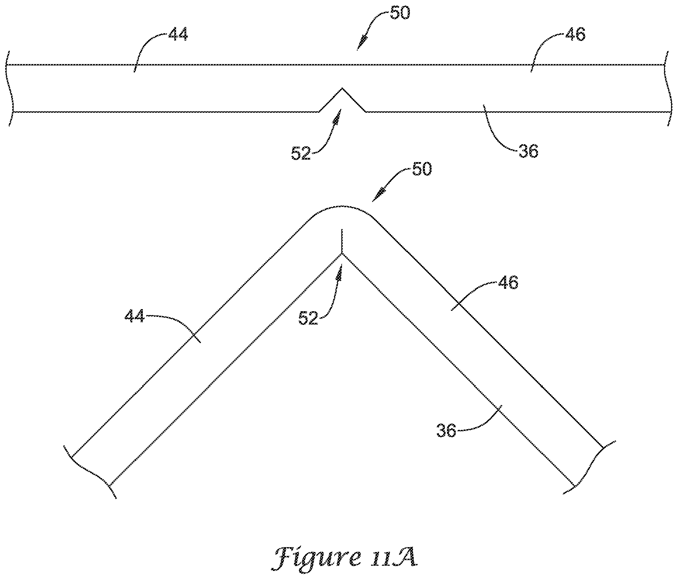

In some embodiments, the outer extent 48 of the struts 36 where the proximal portions 44 bend relative to the distal portions 46 may include a preferred bending location 50. The preferred bending location 50, located at an intermediate location between the proximal end of the strut 36 and the distal end of the strut 36, may preferentially allow the proximal portion 44 of the strut 36 to bend at an angle to the distal portion 46 of the strut 36 at the preferred bending location 50. FIGS. 11A-11C illustrates some possible configurations of the preferred bending location 50 of the struts 36.

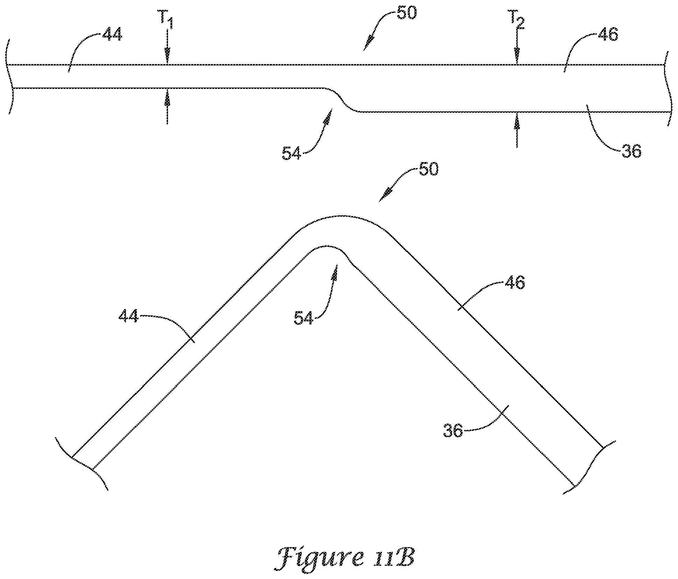

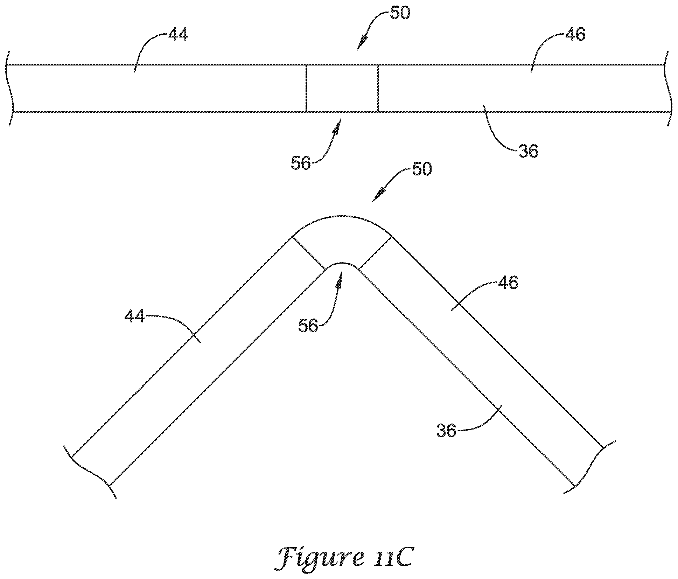

As shown in FIG. 11A, in some instances, the preferred bending location 50 may be a reduced thickness region of the strut 36, such as a notch 52 between the proximal portion 44 and the distal portion 46, creating a reduced thickness region of the strut 36 between the proximal portion 44 and the distal portion 46. The strut 36 may bend at the notch 52, orienting the proximal portion 44 at an angle to the distal portion 46 in the expanded configuration. In other instances, as shown in FIG. 11B, the preferred bending location 50 may be a transition in thickness 54 between the proximal portion 44 and the distal portion 46. For example, the proximal portion 44 may have a thickness T₁ and the distal portion 46 may have a thickness T₂ different from the thickness T₁. In some embodiments, the thickness T₁ may be less than the thickness T₂, while in other embodiments the thickness T₁ may be greater than the thickness T₂, depending on the bending characteristics desired. The strut 36 may bend at the region 54, orienting the proximal portion 44 at an angle to the distal portion 46 in the expanded configuration. The portion of less thickness may more readily bend compared to the portion of greater thickness. As shown in FIG. 11C, in other instances, the preferred bending location 50 may be a region 56 of dissimilar material stiffness than the proximal portion 44 and/or the distal portion 46. For example, the region 56 of dissimilar material stiffness may be formed of a material dissimilar to the material forming the proximal portion 44 and/or the distal portion 46, or the region 56 of dissimilar material stiffness may undergo a manufacturing process, such as annealing, cold working, etc. to impart a different stiffness in the region 56. The strut 36 may bend at the region 56, orienting the proximal portion 44 at an angle to the distal portion 46 in the expanded configuration.

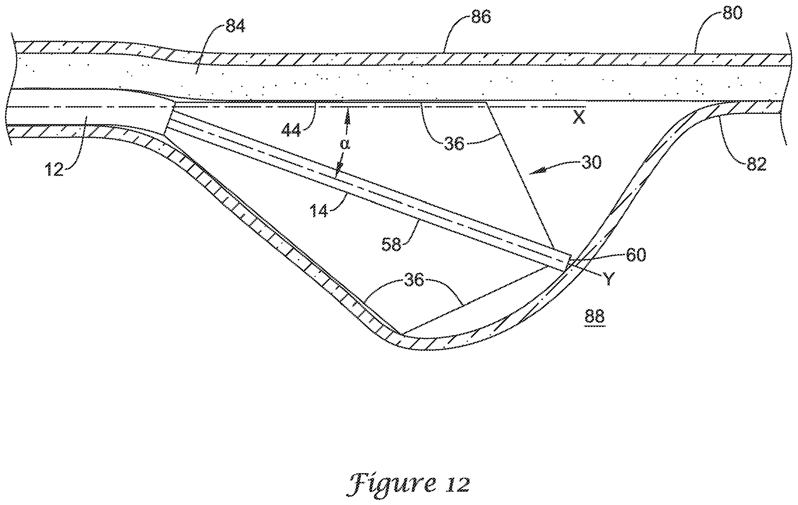

FIG. 12 is a cross-sectional view of the distal portion of the catheter 10 positioned in a subintimal space created between two tissue layers of a vessel wall 80. The blood vessel 80 typically has three tissue layers, an innermost layer or intima layer (i.e., tunica intima) 82, an intermediate layer or media layer (i.e., tunica media) 84, and an outermost layer or adventitia layer (tunica adventitia) 86, with the media layer 84 positioned between the intima layer 82 and the adventitia layer 86. The intima layer 82 is a layer of endothelial cells lining the lumen 88 of the vessel 80, as well as a subendothelial layer made up of mostly loose connective tissue. The media layer 84 is a muscular layer formed primarily of circumferentially arranged smooth muscle cells. The adventitia layer 86, which forms the exterior layer of the vessel wall 80 is formed primarily of loose connective tissue made up of fibroblasts and associated collagen fibers.

As will be described further herein, the distal portion of the catheter 10, including the expandable structure 30 in the collapsed configuration, may be advanced into a subintimal space (i.e., a space between the intima layer 82 and the adventitia layer 86) created in the vessel wall 80, such as through dissection of the tissue layers of the vessel wall 80. Once positioned in the subintimal space, the expandable structure 30 may be expanded to the expanded configuration between the intima layer 82 and the adventitia layer 86 of the vessel wall 80.

The configuration of the expandable structure 30 in the expanded configuration may automatically cause the distal portion 58 of the inner tubular member 14 that extends through the expandable structure 30 to deflect or bend away from the longitudinal axis X of the outer tubular member 12 toward the lumen 88 of the blood vessel 80 within the subintimal space. Since the outer, adventitia layer 86 is stiffer and less compliant than the inner, intima layer 82 of the vessel wall 80, the intima layer 82 will yield first, causing the distal portion 58 of the inner tubular member 14 to bend away from the adventitia layer 86 as the expandable structure 30 is expanded. Accordingly, the proximal portion 44 of the strut 36, or other portion of the expandable structure 30, located between the distal portion 58 of the inner tubular member 14 may remain oriented generally parallel to the adventitia layer 86 while the distal portion 58 of the inner tubular member 14 is deflected or bent away from the longitudinal axis X of the outer tubular member 12 and the adventitia layer 86. Thus, in the expanded configuration, the expandable structure 20 may position the central longitudinal axis Y of the distal portion 58 of the inner tubular member 14 at an angle γ to the central longitudinal axis X of the outer tubular member 12, which is approximately equal to the angle θ₁ that the proximal portion 58 of the strut 36 extends away from the inner tubular member 14. The indicator 38 in the handle or hub assembly 20 may function to indicate to what extent the expandable structure 30 is expanded to, and thus indicate an extent to which the distal portion 58 of the inner tubular member 14 is deflected away from the longitudinal axis X of the outer tubular member 12, and thus the longitudinal axis of a proximal portion of the inner tubular member 14 disposed within the outer tubular member 12. The indicator 38 may provide a visual indication of the extent that the inner tubular member 14 is deflected toward the lumen 88, which may be directly related to the extent to which the expandable structure 30 has expanded from the collapsed configuration to the expanded configuration.

When the expandable structure 30 is expanded, the distal opening of the lumen 18 of the inner tubular member 14 at the distal tip 60 may be oriented toward the lumen 88 such that a distal portion of a penetration member may be advanced distally out of the distal opening to penetrate through the intima layer 82 into the lumen 88 of the blood vessel 80 distal of an occlusion.

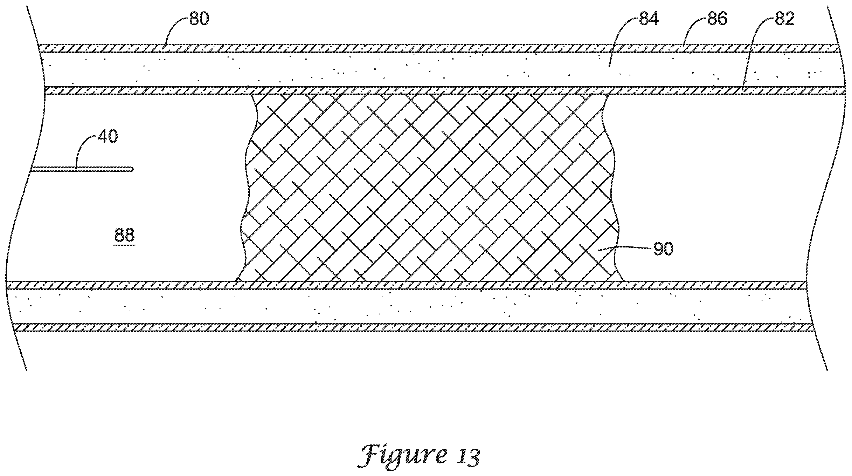

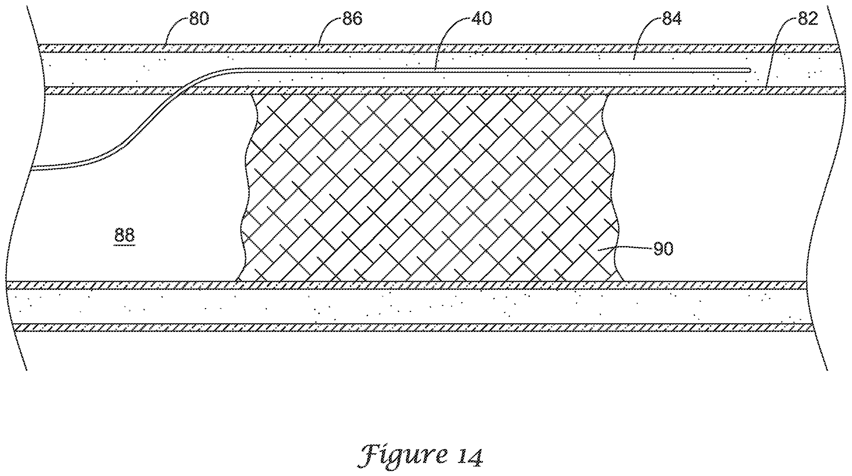

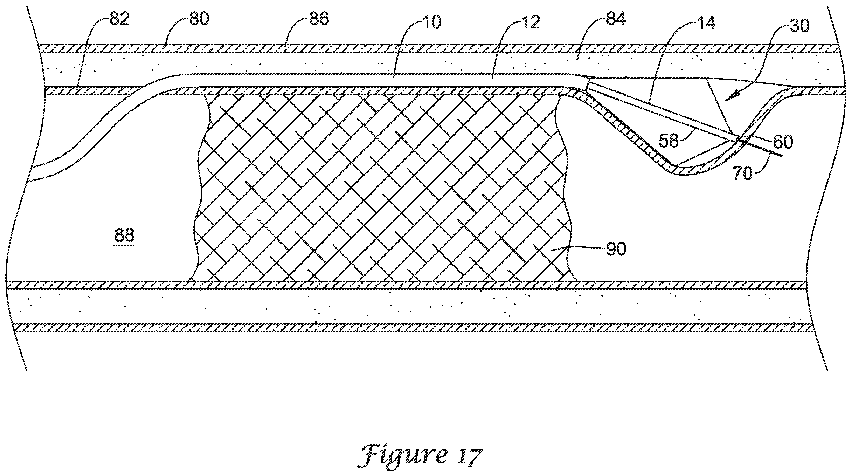

In some instances, it may be undesired, difficult or impossible to pass through an occlusion, such as a chronic total occlusion (CTO) in a lumen of a blood vessel with a medical device to recanalize the vessel. In such instances, it may be possible to recanalize the blood vessel through a subintimal approach using the catheter 10. Turning to FIGS. 13-17, several aspects of an exemplary method for recanalizing an occluded blood vessel using the catheter 10 are illustrated. As shown in FIG. 13, a guidewire 40 may initially be advanced through the lumen 88 of the vessel 80 to a location proximate a proximal end of an occlusion 90 blocking the lumen 88. The guidewire 40 may then be advanced to penetrate outward through the intima layer 82 at a location proximal of the proximal end of the occlusion 90 into the vessel wall 80. With the tip of the guidewire 40 located between the intima layer 82 and the adventitia layer 86, the guidewire 40 may be further advanced distally in a subintimal manner to create a subintimal space between the intima layer 82 and the adventitia layer 86. As shown in FIG. 14, the guidewire 40 may be advanced in a subintimal manner until the distal tip of the guidewire 40 is located distal of the distal end of the occlusion 90 in the subintimal space created, such as by dissection of the tissue layers of the vessel wall 80.

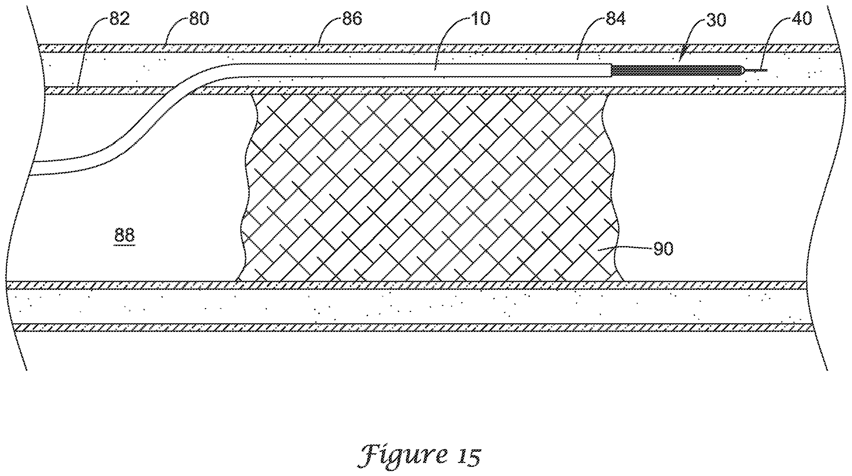

The recanalization catheter 10 may then be advanced distally over the guidewire 40 from the true lumen 88 proximal of the occlusion 90, into the subintimal space between the intima layer 82 and the adventitia layer 86, to a position in the subintimal space in which the distal portion of the catheter 10, including the expandable structure 30, is located distal of the distal end of the occlusion 90, as shown in FIG. 15. The recanalization catheter 10 may be advanced through the subintimal space in a delivery configuration, such as with the expandable structure 30 in the collapsed configuration.

With the expandable structure 30 positioned distal of the distal end of the occlusion 90, the guidewire 40 may be withdrawn proximally such that the distal tip of the guidewire 40 is located proximal of the expandable structure 30. In some instances, the guidewire 40 may be withdrawn completely from the lumen 18 of the inner tubular member 14, while in other instances the guidewire 40 may be retained in a proximal portion of the inner tubular member 14 proximal of the expandable structure 30.

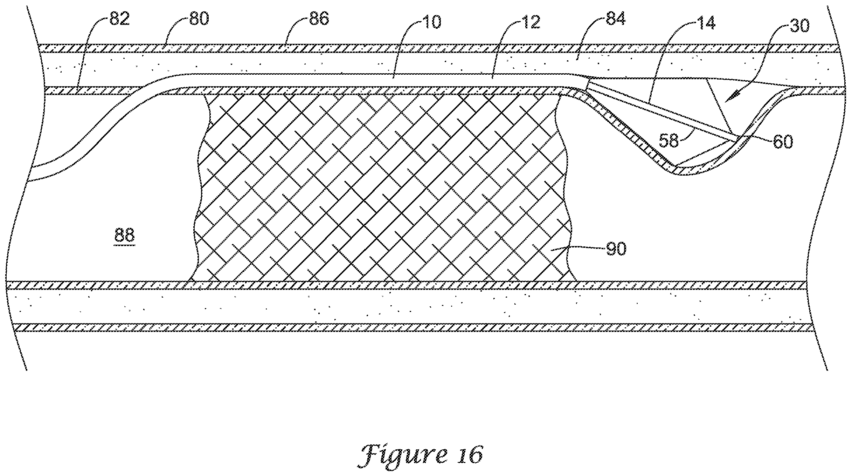

The expandable structure 30 may then be expanded to the expanded configuration in the subintimal space formed between the intima layer 82 and the adventitia layer 86, as shown in FIG. 16. Expanding the expandable structure 30 to or toward the expanded configuration may automatically cause the distal portion 58 of the inner tubular member 14 that extends through the expandable structure 30 to deflect or bend toward the lumen 88 of the blood vessel 80 within the subintimal space to orient the distal opening of the lumen 18 of the inner tubular member 14 at the distal tip 60 toward the lumen 88.

The indicator 38 in the handle or hub assembly 20 may be utilized to indicate to what extent the expandable structure 30 is expanded to, and thus indicate an extent to which the distal portion 58 of the inner tubular member 14 is deflected toward the lumen 88 of the blood vessel 80. Thus, the user may visually observe the indicator 38 at the proximal end of the catheter 10 to visually confirm the amount of longitudinal movement between the inner tubular member 14 and the outer tubular member 12 to provide feedback regarding the extent to which the expandable structure 30 has expanded from the collapsed configuration to the expanded configuration, which may be directly related to the extent that the inner tubular member 14 is deflected toward the lumen 88.

When the expandable structure 30 is expanded, the distal opening of the lumen 18 of the inner tubular member 14 at the distal tip 60 may be oriented toward the lumen 88 such that a distal portion of a penetration member may be advanced distally out of the distal opening to penetrate through the intima layer 82 into the lumen 88 of the blood vessel 80 distal of the occlusion 90.

Once the expandable structure 30 is expanded and the inner tubular member 14 is deflected toward the lumen 88 such that the distal opening of the lumen 18 is oriented toward the lumen 88, a penetration member 70, sized to be advanced through the lumen 18 of the inner tubular member 14, may be advanced through the lumen 18 and distally out of the distal opening of the inner tubular member 14. In some embodiments, the penetration member 70 may be the guidewire 40, or another guidewire introduced through the lumen 18 of the inner tubular member 14. In other embodiments, the penetration member 70 may be an elongate member, such as a needle cannula, having a sharpened distal tip configured to pierce through the intima layer 82 into the lumen 88 distal of the occlusion 90.

In the event the penetration member 70 is a guidewire, the catheter 10 may be withdrawn while leaving the guidewire routed around the occlusion 90 via the subintimal pathway. In instances in which the penetration member 70 is a separate elongate member, such as a needle cannula, the penetration member 70 may be withdrawn and replaced with a guidewire. Thereafter, the catheter may be withdrawn while leaving the guidewire routed around the occlusion 90 via the subintimal pathway.

Once a pathway has been created around the occlusion 90 via a subintimal track, one or more additional medical devices may be advanced through the blood vessel 80 to enlarge the pathway and/or pass distally of the occlusion 90 to perform a further medical procedure.

## Claims

1. A subintimal recanalization catheter assembly for recanalizing a blood vessel having an occlusion in a lumen thereof, the catheter assembly comprising:
an outer tubular member (12; 112) having a lumen (16; 116) extending therethrough;
an inner tubular member (14; 114) having a lumen (18; 118) extending therethrough, the inner tubular member (14; 114) being disposed in the lumen (16; 116) of the outer tubular member (12; 112) and movable relative to the outer tubular member (12; 112);
an expandable structure (30; 130) secured to a distal portion of the inner tubular member (14; 114), the expandable structure (30; 130) configured to be actuated between a collapsed configuration and an expanded configuration by longitudinal movement of the inner tubular member (14; 114) relative to the outer tubular member (12; 112); and
a penetration member (70) sized to be advanced through the lumen (18; 118) of the inner tubular member (14; 114) such that a distal portion of the penetration member (70) is extendable distally of a distal end of the inner tubular member (14; 114);
wherein the catheter assembly is configured to be advanced into a subintimal space between a first tissue layer and a second tissue layer of a wall of the blood vessel to facilitate reentry into the lumen of the blood vessel by advancing the penetration member (70) distally out of the lumen (18; 118) of the inner tubular member (14; 114) and through the first tissue layer into the lumen of the blood vessel distal of the occlusion.

2. The catheter assembly of claim 1, wherein expanding the expandable structure (30; 130) from the collapsed configuration to the expanded configuration causes a distal portion of the catheter assembly to deflect toward the lumen of the blood vessel within the subintimal space.

3. The catheter assembly of claim 2, wherein a distal end of the expandable structure (30; 130) is secured to the inner tubular member (14; 114) and a proximal end of the expandable structure (30; 130) is secured to the outer tubular member (12; 112), wherein longitudinal movement of the inner tubular member (14; 114) proximally relative to the outer tubular member (12; 112) causes the expandable structure (30; 130) to mechanically expand to the expanded configuration.

4. The catheter assembly of claim 3, further comprising a handle assembly (20) including an indicator configured to indicate to what extent the expandable structure (30; 130) is expanded to.

5. The catheter assembly of claim 4, further comprising a locking mechanism (62) to selectively lock the inner tubular member (14; 114) from longitudinal movement relative to the outer tubular member (12; 112) at one of a plurality of positions between the collapsed configuration and the expanded configuration.

6. The catheter assembly of claim 2, wherein the expandable structure (30; 130) includes a first end fixed to the inner tubular member (14; 114) and a second end slidably coupled to the inner tubular member (14; 114), wherein preferably the expandable structure (30; 130) automatically expands to the expanded configuration when unconstrained by the outer tubular member (12; 112).

7. The catheter assembly of claim 1, wherein the expandable structure (30; 130) includes a plurality of struts (36; 136), wherein each of the plurality of struts (36; 136) include a predefined bending location positioned at an intermediate location between a proximal end of the strut (36; 136) and a distal end of the strut (36; 136), wherein a proximal portion of the strut is configured to preferentially bend at an angle to a distal portion of the strut at the predefined bending location.

8. The catheter assembly of claim 7, wherein the predefined bending location is one of:
a reduced thickness region of the strut, a transition in thickness between the proximal portion and the distal portion or a region of dissimilar material stiffness than the proximal and distal portions.

9. The catheter assembly of claim 1, the catheter assembly comprising:
a hub assembly;
the outer tubular member (12; 112) extending distally from the hub assembly;
the inner tubular member (14; 114) extending distally from the hub assembly through the lumen (16; 116) of the outer tubular member (12; 112).

10. The catheter assembly of claim 9, wherein expansion of the expandable structure (30; 130) within the subintimal space causes a distal portion of the inner tubular member (14; 114) extending through the expandable structure (30; 130) to deflect toward the lumen of the blood vessel.

11. The catheter assembly of claim 10, further comprising a handle assembly including an indicator configured to indicate an extent to which the distal portion of the inner tubular member (14; 114) is deflected away from a longitudinal axis of a proximal portion of the inner tubular member (14; 114).

12. The catheter assembly of claim 9, further comprising a handle assembly including an indicator configured to indicate to what extent the expandable structure (30; 130) is expanded to.

13. The catheter assembly of claim 12, further comprising a locking mechanism to selectively lock the inner tubular member (14; 114) from longitudinal movement relative to the outer tubular member (12; 112) at one of a plurality of positions between the collapsed configuration and the expanded configuration.

14. The catheter assembly of claim 9, wherein the inner tubular member (14; 114) is rotatable relative to the outer tubular member (12; 112) to produce longitudinal movement of the inner tubular member (14; 114) relative to the outer tubular member (12; 112).

15. The catheter assembly of claim 9, wherein the expandable structure (30; 130) includes a plurality of longitudinal struts arranged circumferentially about the inner tubular member (14; 114), wherein each of the plurality of longitudinal struts includes a proximal portion extending at an oblique angle to a distal portion of the strut in the expanded configuration, wherein the proximal portion of each strut extends away from the inner tubular member (14; 114) at an angle of between about 30° to about 60° from a longitudinal axis of the inner tubular member (14; 114).

## Patentansprüche

1. Subintimale Rekanalisation-Katheteranordnung zum Rekanalisieren eines Blutgefäßes, das in seinem Lumen eine Okklusion hat, wobei die Katheteranordnung aufweist:
ein Außenrohrelement (12; 112) mit einem hindurchgehenden Lumen (16; 116);
ein Innenrohrelement (14; 114) mit einem hindurchgehenden Lumen (18; 118), wobei das Innenrohrelement (14; 114) in dem Lumen (16; 116) des Außenrohrelements (12; 112) angeordnet ist und relativ zu dem Außenrohrelement (12; 112) bewegbar ist;
eine an einem distalen Abschnitt des Innenrohrelements (14; 114) angebrachte expandierbare Struktur (30; 130), die konfiguriert ist, um durch eine Längsbewegung des Innenrohrelements (14; 114) relativ zu dem Außenrohrelement (12; 112) zwischen einer kollabierten Konfiguration und einer expandierten Konfiguration betätigt zu werden; und
ein Eindringungselement (70), das bemessen ist, um durch das Lumen (18; 118) des Innenrohrelements (14; 114) vorgerückt zu werden, so dass ein distaler Abschnitt des Eindringungselements (70) distal eines distalen Endes des Innenrohrelements (14; 114) ausfahrbar ist;
wobei die Katheteranordnung konfiguriert ist, um in einen subintimalen Raum zwischen einer ersten Gewebeschicht und einer zweiten Gewebeschicht einer Wand des Blutgefäßes vorgerückt zu werden, um einen Wiedereintritt in das Lumen des Blutgefäßes zu erleichtern durch Vorrücken des Eindringungselements (70) distal aus dem Lumen (18; 118) des Innenrohrelements (14; 114) und durch die erste Gewebeschicht in das Lumen des Blutgefäßes distal der Okklusion.

2. Katheteranordnung nach Anspruch 1, wobei ein Expandieren der expandierbaren Struktur (30; 130) aus der kollabierten Konfiguration in die expandierte Konfiguration bewirkt, dass ein distaler Abschnitt der Katheteranordnung innerhalb des subintimalen Raums hin zu dem Lumen des Blutgefäßes abgelenkt wird.

3. Katheteranordnung nach Anspruch 2, wobei ein distales Ende der expandierbaren Struktur (30; 130) an dem Innenrohrelement (14; 114) angebracht ist und ein proximales Ende der expandierbaren Struktur (30; 130) an dem Außenrohrelement (12; 112) angebracht ist, wobei eine Längsbewegung des Innenrohrelements (14; 114) proximal relativ zu dem Außenrohrelement (12; 112) ein mechanisches Expandieren der expandierbaren Struktur (30; 130) in die expandierte Konfiguration bewirkt.

4. Katheteranordnung nach Anspruch 3, ferner aufweisend eine Griffanordnung (20) mit einem Indikator, der konfiguriert ist, um anzuzeigen, in welchem Ausmaß die expandierbare Struktur (30; 130) expandiert ist.

5. Katheteranordnung nach Anspruch 4, ferner aufweisend einen Verriegelungsmechanismus (62) zum selektiven Verriegeln einer Längsbewegung des Innenrohrelements (14; 114) relativ zu dem Außenrohrelement (12; 112) an einer von mehreren Positionen zwischen der kollabierten Konfiguration und der expandierten Konfiguration.

6. Katheteranordnung nach Anspruch 2, wobei die expandierbare Struktur (30; 130) ein an dem Innenrohrelement (14; 114) angebrachtes erstes Ende und ein gleitbar mit dem Innenrohrelement (14; 114) gekoppeltes zweites Ende aufweist, wobei die expandierbare Struktur (30; 130) vorzugsweise automatisch in die expandierte Konfiguration expandiert, wenn sie nicht von dem Außenrohrelement (12; 112) behindert wird.

7. Katheteranordnung nach Anspruch 1, wobei die expandierbare Struktur (30; 130) mehrere Streben (36; 136) aufweist, wobei jede der mehreren Streben (36; 136) einen vorgegebenen Biegeort aufweist, der sich an einem mittleren Ort zwischen einem proximalen Ende der Strebe (36; 136) und einem distalen Ende der Strebe (36; 136) befindet, wobei ein proximaler Abschnitt der Strebe konfiguriert ist, um sich vorzugsweise an dem vorgegebenen Biegeort in einem Winkel zu einem distalen Abschnitt der Strebe zu biegen.

8. Katheteranordnung nach Anspruch 7, wobei der vorgegebene Biegeort einer ist von:
ein in der Dicke reduzierter Bereich der Strebe, ein Übergang in der Dicke zwischen dem proximalen Abschnitt und dem distalen Abstand oder ein Bereich anderer Materialsteifheit als der proximale und distale Abschnitt.

9. Katheteranordnung nach Anspruch 1, wobei die Katheteranordnung aufweist:
eine Nabenanordnung;
das Außenrohrelement (12; 112), das sich distal von der Nabenanordnung erstreckt;
das Innenrohrelement (14; 114), das sich distal von der Nabenanordnung durch das Lumen (16; 116) des Außenrohrelements (12; 112) erstreckt.

10. Katheteranordnung nach Anspruch 9, wobei ein Expandieren der expandierbaren Struktur (30; 130) innerhalb des subintimalen Raums bewirkt, dass ein sich durch die expandierbare Struktur (30; 130) ersteckender distaler Abschnitt des Innenrohrelements (14; 114) hin zu dem Lumen des Blutgefäßes abgelenkt wird.

11. Katheteranordnung nach Anspruch 10, ferner aufweisend eine Griffanordnung mit einem Indikator, der konfiguriert ist, um ein Ausmaß anzuzeigen, in welchem der distale Abschnitt des Innenrohrelements (14; 114) weg von einer Längsachse eines proximalen Abschnitts des Innenrohrelements (14; 114) abgelenkt ist.

12. Katheteranordnung nach Anspruch 9, ferner aufweisend eine Griffanordnung mit einem Indikator, der konfiguriert ist, um anzuzeigen, in welchem Ausmaß die expandierbare Struktur (30; 130) expandiert ist.

13. Katheteranordnung nach Anspruch 12, ferner aufweisend einen Verriegelungsmechanismus zum selektiven Verriegeln einer Längsbewegung des Innenrohrelements (14; 114) relativ zu dem Außenrohrelement (12; 112) an einer von mehreren Positionen zwischen der kollabierten Konfiguration und der expandierten Konfiguration.

14. Katheteranordnung nach Anspruch 9, wobei das Innenrohrelement (14; 114) relativ zu dem Außenrohrelement (12; 112) drehbar ist, um eine Längsbewegung des Innenrohrelements (14; 114) relativ zu dem Außenrohrelement (12; 112) zu erzeugen.

15. Katheteranordnung nach Anspruch 9, wobei die expandierbare Struktur (30; 130) mehrere Längsstreben aufweist, die im Umkreis um das Innenrohrelement (14; 114) angeordnet sind, wobei jede der mehreren Längsstreben einen proximalen Abschnitt aufweist, der in der expandierten Konfiguration sich in einem schrägen Winkel zu einem distalen Abschnitt der Strebe erstreckt, wobei der proximale Abschnitt jeder Strebe sich weg von dem Innenrohrelement (14; 114) erstreckt, um mit einer Längsachse des Innenrohrelements (14; 114) einen Winkel zwischen ungefähr 30° und ungefähr 60° zu bilden.

## Revendications

1. Ensemble formant cathéter de recanalisation sous-intimale pour recanaliser un vaisseau sanguin ayant une occlusion dans une lumière de celui-ci, l'ensemble formant cathéter comprenant :
un membre tubulaire externe (12 ; 112) ayant une lumière (16 ; 116) s'étendant à travers celui-ci ;
un membre tubulaire interne (14 ; 114) ayant une lumière (18 ; 118) s'étendant à travers celui-ci, le membre tubulaire interne (14 ; 114) étant disposé dans la lumière (16 ; 116) du membre tubulaire externe (12 ; 112) et déplaçable par rapport au membre tubulaire externe (12 ; 112) ;
une structure expansible (30 ; 130) immobilisée sur une partie distale du membre tubulaire interne (14 ; 114), la structure expansible (30 ; 130) configurée pour être actionnée entre une configuration affaissée et une configuration expansée par un mouvement longitudinal du membre tubulaire interne (14 ; 114) par rapport au membre tubulaire externe (12 ; 112) ; et
un membre de pénétration (70) dimensionné pour être avancé à travers la lumière (18 ; 118) du membre tubulaire interne (14 ; 114) de sorte qu'une partie distale du membre de pénétration (70) peut s'étendre distalement d'une extrémité distale du membre tubulaire interne (14 ; 114) ;
où l'ensemble formant cathéter est configuré pour être avancé dans un espace sous-intimal entre une première couche de tissu et une seconde couche de tissu d'une paroi du vaisseau sanguin pour faciliter la rentrée dans la lumière du vaisseau sanguin en avançant le membre de pénétration (70) distalement hors de la lumière (18 ; 118) du membre tubulaire interne (14 ; 114) et à travers la première couche de tissu dans la lumière du vaisseau sanguin distalement de l'occlusion.

2. Ensemble formant cathéter selon la revendication 1, où l'expansion de la structure expansible (30 ; 130) depuis la configuration affaissée jusque dans la configuration expansée amène une partie distale de l'ensemble formant cathéter à s'incurver en direction de la lumière du vaisseau sanguin dans l'espace sous-intimal.

3. Ensemble formant cathéter selon la revendication 2, où une extrémité distale de la structure expansible (30 ; 130) est immobilisée sur le membre tubulaire interne (14 ; 114) et une extrémité proximale de la structure expansible (30 ; 130) est immobilisée sur le membre tubulaire externe (12 ; 112), où un mouvement longitudinal du membre tubulaire interne (14 ; 114) proximalement par rapport au membre tubulaire externe (12 ; 112) amène la structure expansible (30 ; 130) à s'expanser mécaniquement jusque dans la configuration expansée.

4. Ensemble formant cathéter selon la revendication 3, comprenant en outre un ensemble formant poignée (20) incluant un indicateur configuré pour indiquer dans quelle mesure la structure expansible (30 ; 130) est expansée.

5. Ensemble formant cathéter selon la revendication 4, comprenant en outre un mécanisme de verrouillage (62) pour verrouiller sélectivement le membre tubulaire interne (14 ; 114) contre un mouvement longitudinal par rapport au membre tubulaire externe (12 ; 112) dans l'une d'une pluralité de positions entre la configuration affaissée et la configuration expansée.

6. Ensemble formant cathéter selon la revendication 2, où la structure expansible (30 ; 130) inclut une première extrémité fixée au membre tubulaire interne (14 ; 114) et une seconde extrémité couplée à coulissement au membre tubulaire interne (14 ; 114), où de préférence la structure expansible (30 ; 130) est expansée automatiquement jusque dans la configuration expansée quand elle n'est pas contrainte par le membre tubulaire externe (12 ; 112).

7. Ensemble formant cathéter selon la revendication 1, où la structure expansible (30 ; 130) inclut une pluralité d'étais (36 ; 136), où chacun de la pluralité d'étais (36 ; 136) inclut un emplacement de flexion prédéfini positionné à un emplacement intermédiaire entre une extrémité proximale de l'étai (36 ; 136) et une extrémité distale de l'étai (36 ; 136), où une partie proximale de l'étai est configurée pour fléchir préférentiellement sous un angle avec une partie distale de l'étai à l'emplacement de flexion prédéfini.

8. Ensemble formant cathéter selon la revendication 7, où l'emplacement de flexion prédéfini est l'un de : une région d'épaisseur réduite de l'étai, une transition dans l'épaisseur entre la partie proximale et la partie distale ou une région de rigidité de matériau dissimilaire par rapport aux parties proximale et distale.

9. Ensemble formant cathéter selon la revendication 1, l'ensemble formant cathéter comprenant :
un ensemble formant moyeu ;
le membre tubulaire externe (12 ; 112) s'étendant distalement depuis l'ensemble formant moyeu ;
le membre tubulaire interne (14 ; 114) s'étendant distalement depuis l'ensemble formant moyeu à travers la lumière (16 ; 116) du membre tubulaire externe (12 ; 112).

10. Ensemble formant cathéter selon la revendication 9, où l'expansion de la structure expansible (30 ; 130) dans l'espace sous-intimal amène une partie distale du membre tubulaire interne (14 ; 114) s'étendant à travers la structure expansible (30 ; 130) à s'incurver en direction de la lumière du vaisseau sanguin.

11. Ensemble formant cathéter selon la revendication 10, comprenant en outre un ensemble formant poignée incluant un indicateur configuré pour indiquer une mesure dans laquelle la partie distale du membre tubulaire interne (14 ; 114) est incurvée en s'éloignant d'un axe longitudinal d'une partie proximale du membre tubulaire interne (14 ; 114).

12. Ensemble formant cathéter selon la revendication 9, comprenant en outre un ensemble formant poignée incluant un indicateur configuré pour indiquer dans quelle mesure la structure expansible (30 ; 130) est expansée.

13. Ensemble formant cathéter selon la revendication 12, comprenant en outre un mécanisme de verrouillage pour verrouiller sélectivement le membre tubulaire interne (14 ; 114) contre un mouvement longitudinal par rapport au membre tubulaire externe (12 ; 112) dans l'une d'une pluralité de positions entre la configuration affaissée et la configuration expansée.

14. Ensemble formant cathéter selon la revendication 9, où le membre tubulaire interne (14 ; 114) peut tourner par rapport au membre tubulaire externe (12 ; 112) pour produire un mouvement longitudinal du membre tubulaire interne (14 ; 114) par rapport au membre tubulaire externe (12 ; 112).

15. Ensemble formant cathéter selon la revendication 9, où la structure expansible (30 ; 130) inclut une pluralité d'étais longitudinaux disposés de manière circonférentielle autour du membre tubulaire interne (14 ; 114), où chacun de la pluralité d'étais longitudinaux inclut une partie proximale s'étendant sous un angle oblique par rapport à une partie distale de l'étai dans la configuration expansée, où la partie proximale de chaque étai s'étend en s'éloignant du membre tubulaire interne (14 ; 114) sous un angle d'entre environ 30° et environ 60° avec un axe longitudinal du membre tubulaire interne (14 ; 114).
